# EUROPEAN PATENT SPECIFICATION

(11) **EP 3 207 945 B1**
(45) Date of publication and mention of the grant of the patent: **08.09.2021**
(21) Application number: 15850079.3
(22) Date of filing: 21.07.2015
(51) Int. Cl.: A61K 9/22, A61K 31/155

(54) **SUSTAINED RELEASE FORMULATION OF METFORMIN AND METHOD FOR PREPARING SAME**
METFORMINFORMULIERUNG MIT VERZÖGERTER FREISETZUNG UND VERFAHREN ZUR HERSTELLUNG DAVON
FORMULATION DE METFORMINE À LIBÉRATION PROLONGÉE ET PROCÉDÉ POUR SA PRÉPARATION

(30) Priority: 13.10.2014 KR 20140137815
(43) Date of publication of application: 23.08.2017
(73) Proprietor: CJ Healthcare Corporation, Jung-gu, Seoul 100-400 (KR)
(72) Inventor: PARK, Jun Hong, Yongin-si Gyeonggi-do 449-060 (KR); LEE, Ji Eun, Incheon 405-837 (KR); KIM, Yu Jeong, Seoul 156-827 (KR); LYU, Chun Seon, Yongin-si Gyeonggi-do 446-956 (KR); SEO, Hyuk Seong, Seoul 134-737 (KR); OH, Tack Oon, Gwangju-si Gyeonggi-do 464-925 (KR); JEON, Eun Kyung, Hwaseong-si Gyeonggi-do 445-733 (KR); HAN, Sung Kyun, Hwaseong-si Gyeonggi-do 445-792 (KR)
(74) Representative: SONN Patentanwälte OG
(86) International application number: PCT/KR2015/007526
(87) International publication number: WO 2016/060365

(56) References cited:
- KR-A- 20060 096 728
- KR-B1- 100 791 844
- KR-B1- 100 791 844
- None

## Description

### Technical Field

The present invention relates to a gastric-retention type sustained-release formulation comprising metformin, and a preparation method thereof.

### Background Art

Metformin is a therapeutic agent for insulin-independent diabetes, and chemically has a biguanide structure. Metformin controls glucose production in the gastrointestinal tract and increases the glucose utilization rate in muscles, and thus has been used for the prevention and treatment of diabetes. Additionally, metformin has advantages in that it can improve lipid metabolism, thus preventing body weight increase, and can be used for the prevention and treatment of occurrence and worsening of diabetes complications (e.g., cardiovascular diseases, etc.).

However, because of the high water solubility of metformin, when metformin is formulated into a tablet, it may cause an excessive drop in blood glucose level due to its rapid release, thereby causing a gastrointestinal disorder. Additionally, metformin is conventionally administered in as much as from 500 mg to 850 mg two or three times daily as a fast-release tablet (2,550 mg of daily maximum), and thus the rapid change in blood glucose level due to its rapid release may aggravate adverse actions and resistance to metformin. Furthermore, since metformin has a short half-life of 2 hours to 6 hours in blood, it is desirable to design metformin to be administered as a once-daily sustained-release formulation to improve therapeutic effects as well as to improve patient compliance.

The conventional sustained-release metformin formulations used for once-daily administration, although they may have accomplished a uniform controlled-release via pharmaceutically well-known technologies, have problems in that the amount of carriers required for sustained-release is too high, and that they are difficult to apply to actual production from a commercial point of view.

International Publication No. WO 1999/47125 discloses a sustained-release technique based on the principle of osmosis using a semi-permeable membrane, but this method has problems in that it is difficult to prepare a uniform semi-permeable membrane, and that there may be a change in drug release depending on the gastric conditions.

International Publication No. WO 1999/47128 discloses a technique for biphasic controlled-release formulations with prolonged gastric residence using an ionic polymer and a nonionic polymer. However, this method requires a large amount of sustained-release agents, thus making the size of the formulations larger and thereby worsening convenience of administration. International Publication No. WO 2002/36100 discloses a technique for controlled-release formulations which controls drug release by laser-drilling of tablets coated with a sustained-release film. However, this method has little industrial value because of the requirement for the use of the expensive device. Additionally, International Publication No. WO 2003/28704 discloses a technique for sustained-release formulations which achieves sustained-release by controlling the water content of the formulations, but this method has a problem in that it is difficult to establish processing conditions to supply a constant amount of water.

Meanwhile, many studies have been performed on sustained-release formulations which slowly release drugs and thereby reduce the frequency of drug administration from the aspect of improving drug compliance. However, regardless of the prolonged drug release, the pharmaceutical efficacy may not be sustained in some drugs, for example, those with a narrow absorption window such as metformin. When the site for drug absorption, after administration, is limited to the upper intestinal tract it will be difficult to obtain the desired pharmaceutical effect if the drug is released after passing through the upper intestinal tract. Accordingly, it is important to control the time that the drug will pass through the upper intestinal tract, and for this purpose, studies have been focused on gastric-retention type sustained-release formulations. The gastric-retention type sustained-release formulations can be largely classified into three classes according to the method of gastric retention; i.e., 'a bioadhesive system', in which a drug is adhered in the body and retained in the stomach; 'a buoyancy system', in which the density of a formulation is reduced using a foaming agent, thereby allowing the drug to be floated within the stomach; and 'an expansion system', in which a drug becomes considerably swollen in the stomach so that the drug cannot pass through the pylorus. Among them, the expansion system is in general most commercialized, and swellable polymers with high molecular weight and high viscosity should be contained in a certain amount for its formulation.

Generally, swellable agents for sustained-release, which are hydrophilic and highly viscous, have a three-dimensional network structure, in which they are densely bonded physically or chemically, and thus they become swollen and form a hydrated gel within a short period of time when they are brought into contact with an aqueous solution, thereby preventing the immediate release of drug.

Accordingly, the gastric-retention type sustained-release formulation utilizing swellable agents should be swollen within a short period of time to obtain a size sufficient to be prevented from being passed through the pylorus with a size of 1.2±0.7 cm. Using the swellable polymers alone, it is possible to achieve the target swelling speed caused by rapid hydrogel formation. However, a large amount of swelling agent is required due to high unit dosage and high solubility of metformin, resulting in the increase of tablet size.

Korean Patent Nos. 10-0791844, 10-0858848, and 10-1043816 disclose methods of effectively controlling drug release using a small amount of agents by a concurrent use of the swellable polymer with a water-insoluble agent. The combined use of both agents can control the speed of drug release, but the speed of hydrogel formation of the swellable polymer is lowered by the hydrophobic water-insoluble agent, thereby decreasing the initial swelling rate.

Accordingly, the present inventors, while endeavoring to develop a gastric-retention type sustained-release formulation capable of effectively controlling release of metformin, a hydrophilic drug whose absorption site is limited to the upper intestinal tract due to its narrow absorption window, discovered that a sustained-release formulation comprising a sustained release granuled material, which comprises granules comprising metformin or a pharmaceutically acceptable salt thereof and a swellable polymer and a water-insoluble polymer film coated on the surface of the granules; and a superdisintegrant, can secure a stable release of the active ingredients using a minimum amount of a polymer. The present invention improves convenience of administration by reducing the formulation size, and also simultaneously controls wettability of tablet formulations, thereby completing the present invention.

### Technical Problem

The present invention relates to a gastric-retention type sustained-release formulation of metformin, a hydrophilic drug whose absorption site is limited to the upper intestinal tract due to its narrow absorption window, which can secure gastric-retention time for a stable release and absorption of active ingredients using a minimum amount of a polymer, improve convenience of administration due to its small size, and control wettability of its tablet formulation to be expanded rapidly, and a method of preparing the same.

### Technical Solution

In order to achieve the above objects, in an aspect, the present invention provides a gastric-retention type sustained-release formulation comprising: a sustained-release granulated material, which comprises granules comprising metformin or a pharmaceutically acceptable salt thereof and a swellable polymer, and a coating layer containing a water-insoluble polymer applied on the surface of the granules; and a superdisintegrant, wherein the superdisintegrant is present as an extragranular component mixed with the sustained-release granulated material, wherein the superdisintegrant is at least one selected from the group consisting of croscarmellose sodium and crospovidone, and wherein the superdisintegrant is comprised in the amount from 0.5 wt% to 5 wt% based on the total amount of the formulation.

As used herein, the term "a sustained-release granulated material" refers to a composition comprising metformin or a pharmaceutically acceptable salt thereof, which can prevent rapid release and allow a continuous release of the same. For the sustained release, metformin or a pharmaceutically acceptable salt thereof is granulated along with a swellable polymer, and then the individual granule is coated with a water-insoluble polymer film.

As used herein, the term "metformin" refers to a compound used for the prevention or treatment of insulin-independent diabetes, having a chemical name of N,N-dimethylimidodicarbonimidic diamide (Formula 1 below).

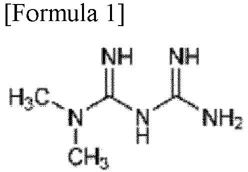

Metformin may be used by separation from natural resources, by manufacturing via chemical modification after obtaining it from natural resources, or easily by a chemical synthesis according to a known method by a skilled person in the art. Alternatively, a commercially available metformin may be purchased for use.

Preferably, metformin or a pharmaceutically acceptable salt thereof may be contained in the sustained-release formulation of the present invention in the amount from 250 mg to 1000 mg.

As used herein, the term "a pharmaceutically acceptable salt" refers to a salt formed with any organic or inorganic acid or base, which does not cause severe irritation to an organism to be administered, or damage the biological activities and physical properties of the chemical. The pharmaceutically acceptable salt may include acid addition salts which can form non-toxic acid addition salts containing pharmaceutically acceptable anions, for example, inorganic acids such as hydrochloric acid, sulfuric acid, nitric acid, phosphoric acid, hydrobromic acid, and hydriodic acid; organic carbonic acids such as tartaric acid, formic acid, citric acid, acetic acid, trichloroacetic acid, trifluoroacetic acid, succinic acid, gluconic acid, benzoic acid, lactic acid, fumaric acid, maleic acid, and salicylic acid; sulfonic acids such as methanesulfonic acid, ethanesulfonic acid, benzenesulfonic acid, and p-toluenesulfonic acid, etc. Additionally, the pharmaceutically acceptable salt may also include metal salts or alkali earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl)methylamine, diethanolamine, choline, and triethylamine, etc. Preferably, the pharmaceutically acceptable salt of metformin may include metformin hydrochloride, acetate, succinate, fumarate, methanesulfonate, beneznesulfonate, toluenesulfonate, etc., but is not limited thereto.

As used herein, the term "a swellable polymer" refers to a pharmaceutically acceptable polymer which becomes swollen in an aqueous solution, thereby capable of controlling drug release. In the present invention, the swellable polymer forms granules along with metformin or a pharmaceutically acceptable salt thereof, and exhibits the sustained-release characteristic of the same. The swellable polymer that can be used in the present invention may include at least one selected from the group consisting of hypromellose, hydroxyethyl cellulose, hydroxypropyl cellulose, polyethylene oxide, carrageenan, natural gum, guar gum, tragacanth, acacia gum, locust bean gum, xanthan gum, polyvinyl alcohol, and polyvinylpyrrolidone, and preferably, hypromellose or polyethylene oxide, but is not limited thereto as long as it is a pharmaceutically acceptable swellable polymer enabling a controlled release according to the purposes of the present invention. Preferably, the swellable polymer has a viscosity of 100 mPas or higher.

Preferably, the swellable polymer may be contained in the amount from 10 wt% to 40 wt% based on the total weight of a given formulation. When the swellable polymer is contained at less than 10 wt%, it becomes difficult to achieve an effective control of drug release, whereas when the swellable polymer is contained at more than 40 wt%, the size of tablets becomes too large to be administered, and is thus not appropriate.

The granules of the present invention comprising metformin or a pharmaceutically acceptable salt thereof and the swellable polymer are formed by coating the external surface with a water-insoluble polymer.

As used herein, the term "a water-insoluble polymer" refers to a pharmaceutically acceptable polymer capable of controlling drug release, which is water-insoluble or hardly soluble in water. Additionally, the purposes of the water-insoluble polymer of the present invention are to prevent the release of metformin or a pharmaceutically acceptable salt thereof, and also to prevent the decrease in viscosity of the swellable polymer with high molecular weight being degraded by oxidation. That is, the formulation according to the present invention is formed such that the swellable polymer can be prevented from physical contact with water, heat, light, etc., and chemical reaction, because of the water-insoluble polymer film coating.

The water-insoluble polymer that can be used in the present invention may include at least one selected from the group consisting of methacrylic acid copolymer, ethylcellulose, hydroxypropyl methylcellulose phthalate, hydroxypropyl methylcellulose acetate succinate, cellulose acetate phthalate, fatty acids, fatty acid esters, fatty acid alcohols, and waxes, and preferably, methacrylic acid copolymer or ethylcellulose, but is not limited thereto as long as it is a pharmaceutically acceptable water-insoluble polymer enabling a controlled release according to the purposes of the present invention.

Preferably, the water-insoluble polymer according to the present invention may be comprised in the amount from 1 wt% to 20 wt% based on the total weight of the formulation. When the polymer content exceeds 20 wt%, it will increase the film thickness and slow hydration of the swellable polymer, and is thus not suitable for controlling the initial release of drug.

According to an exemplary embodiment of the present invention, the sustained-release formulation (Example 1), which was prepared by coating metformin hydrochloride and the swellable polymer together with a water-insoluble polymer film, showed more effectively controlled release profile with a lower release rate compared to the sustained-release formulation (Table 5), which was prepared by coating metformin hydrochloride with water-insoluble polymer then by mixing the metformin hydrochloride granules with a hydrophilic swellable polymer(Comparative Example 5). This result is because, in the formulation of Comparative Example 5, water rapidly penetrates into the interior of the tablet, thereby reducing the effect of the water-insoluble coating membrane.

The sustained-release formulation of the present invention comprises the sustained-release granulated material and a superdisintegrant.

As used herein, the term "a superdisintegrant" generally refers to a material which exhibits a disintegrating effect at low concentrations (2% to 4%), but, according to its use, it may refer to a material that, as a water-insoluble gelling agent, is able to swell and control the release of drug by forming a viscous mixture which delays the diffusion of a drug when it is exposed to water.

Superdisintegrants are used to promote disintegration or disruption of formulations, and thus formulations utilizing a superdisintegrant become rapidly disintegrated. Therefore, superdisintegrants are not suitable for use in sustained-release formulations, which require a slow release of drugs over a long period of time. However, the sustained-release formulation of the present invention was prepared by mixing a superdisintegrant with a sustained-release granulated material so that it enables a sustained release of a drug while securing the initial swelling of the drug at the initial stage in the sustained-release granulated material. That is, in the sustained-release granulated material of the present invention, a water-insoluble polymer film was formed on the exterior of a swellable polymer, thereby effectively controlling the release of a drug, while solving the problem of time delay in hydration of a polymer due to a water-insoluble polymer film, by promoting or accelerating the initial swelling of the swellable polymer by the superdisintegrant, which was mixed with the swellable polymer granule.

The superdisintegrants to be used in the present invention may be at least one selected from the group consisting of croscarmellose sodium and crospovidone. Disclosed are also superdisintegrants of at least one selected from the group consisting of croscarmellose sodium, sodium starch glycolate, and crospovidone, but are not limited thereto, as long as they are pharmaceutically acceptable additives to be used according to the purposes of the present invention.

The superdisintegrant of the present invention may be comprised in the amount from 0.5 wt% to 5 wt%. When the superdisintegrant content exceeds 5 wt%, it is not possible to control wettability of a given formulation, and it is also not suitable for controlling the initial release of a drug.

According to an exemplary embodiment of the present invention, the sustained-release formulations (Examples 2 to 5), which were prepared by mixing a superdisintegrant such as croscarmellose sodium or crospovidone with a sustained-release granulated material prepared by coating metformin hydrochloride and a swellable polymer together with a water-insoluble polymer film, showed a slight decrease or no change in the release rate (Table 7), compared to the sustained-release formulation, not using a superdisintegrant, prepared in Example 1, but further promoted the initial expansion of the formulations. Because the mass of the tablets prepared in Examples corresponded to about 80% of 500 mg Glucophage XR tablets which have a tablet mass exceeding 1,000 mg, there was a difference in their initial volume. However, due to the high expansion rate, the tablets began to expand to be similar or greater in volume compared to 500 mg Glucophage XR tablets since the 2 hour time point and thereafter (Table 8), and thus they could secure a sufficient gastric-retention time necessary for stable release and absorption of the active ingredients while using a lesser amount of a polymer, and improve convenience of administration due to their small size.

Additionally, the sustained-release formulation of the present invention may further comprise a film layer on the external surface. The film layer may be, for example, a shield film layer, a moisture-proofing film layer, or a glucose film layer, etc. Preferably, the external film layer is formed of a water-soluble material, and the material to be used for the water-soluble film layer may be hypromellose, hydroxypropyl cellulose, hydroxyethyl cellulose, cellulose acetate phthalate, ethylcellulose, methylcellulose, polymethacrylate, polyvinyl alcohol-polyethylene glycol graft copolymer (Kollicoat®; BASF, Germany), polyvinyl alcohol (Opadry®; Colorcon, USA), or a combination thereof, but is not limited thereto.

Additionally, the sustained-release formulation of the present invention may be prepared by further using additives conventionally used in the art within the scope of not damaging the effects of the present invention, such as a diluent, a binder, a lubricant, a pH adjuster, an antifoaming agent, a dissolution adjuvant, an antioxidant, etc.

The sustained-release formulation of the present invention may be prepared in various formulation types, for example, tablets such as plain tablets, film-coated tablets, single-layer tablets, double-layer tablets, multi-layer tablets or core tablets, powders, granules, capsules, etc.

The thus-prepared sustained-release formulation of the present invention can provide an appropriate release feature as pharmaceutical active ingredients by continuously releasing a metformin ingredient during in vivo administration. Additionally, a gastric-retention type sustained-release formulation was designed such that convenience of administration was improved by reducing the contents of sustained-release agents necessary for sustained release of metformin, whereas the wettability of the formulation was adjusted to make the formulation become rapidly swollen by including a swellable polymer and a superdisintegrant. Accordingly, the sustained-release formulation of the present invention can be effectively used for the prevention and treatment of diabetes and diabetes complications.

In another aspect, the present invention provides a method of preparing the sustained-release formulation comprising:
(a) preparing granules comprising metformin or a pharmaceutically acceptable salt thereof; and a swellable polymer
(b) preparing a sustained-release granulated material, wherein a coating layer is formed thereon by coating the granules with a water-insoluble polymer; and
(c) preparing a formulation by mixing the sustained-release granulated material and a superdisintegrant.

Additionally, the method of the present invention may further comprise forming a film layer on the external surface of the sustained-release formulation.

As used herein, the term, "metformin", "a swellable polymer"', "a water-insoluble polymer", "a sustained-release granulated material", "a superdisintegrant", "a pharmaceutically acceptable salt", and "a film layer" are the same as described above.

### Advantageous Effects

The gastric-retention type sustained-release formulation of metformin according to the present invention can effectively control drug release using a minimum amount of a polymer by coating the granulated material containing a swellable polymer with a water-insoluble polymer, and also can prevent decrease in viscosity by oxidation or degradation of the swellable polymer structure.

The thus-prepared sustained-release granulated material may cause a delay in hydration of the polymer by forming a water-insoluble polymer film on the external surface of the swellable polymer. In order to remedy this, a superdisintegrant was mixed with the sustained-release granulated material to control the wettability of the formulation.

Accordingly, the present invention has the effect of providing a gastric-retention type sustained-release formulation, which has improved convenience of administration by reducing the amount of sustained-release agents required for sustained release of metformin, and controlled wettability enabling a rapid swelling of the formulation despite its small tablet size.

### Description of Drawings

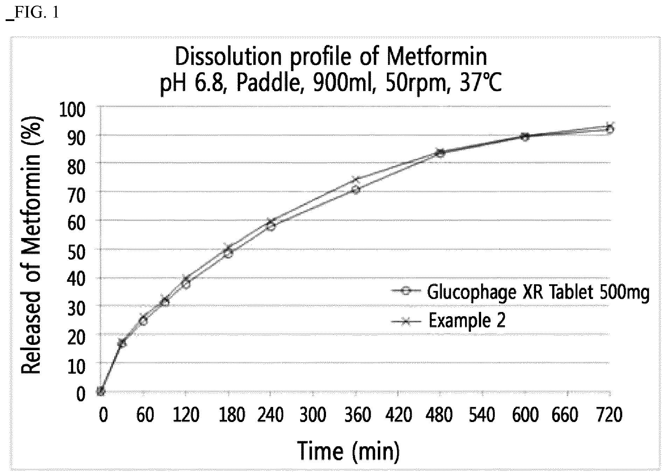

FIG. 1 is a graph illustrating the results of a release experiment on a sustained-release formulation of metformin hydrochloride prepared in Example 2 of the present invention and 500 mg Glucophage XR tablets.

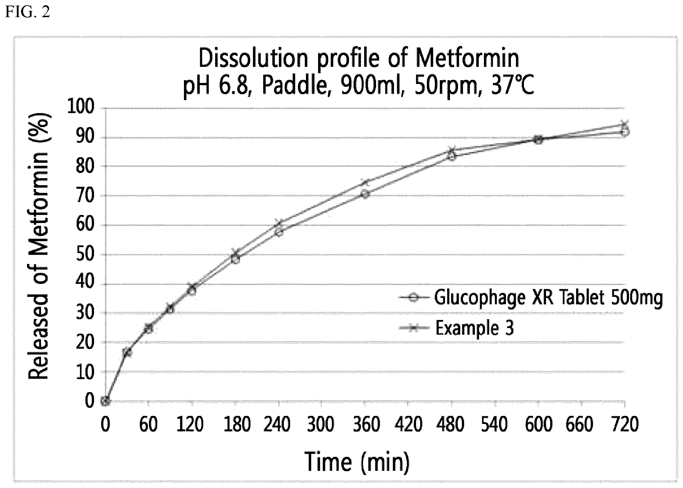

FIG. 2 is a graph illustrating the results of a release experiment on a sustained-release formulation of metformin hydrochloride prepared in Example 3 of the present invention and 500 mg Glucophage XR tablets.

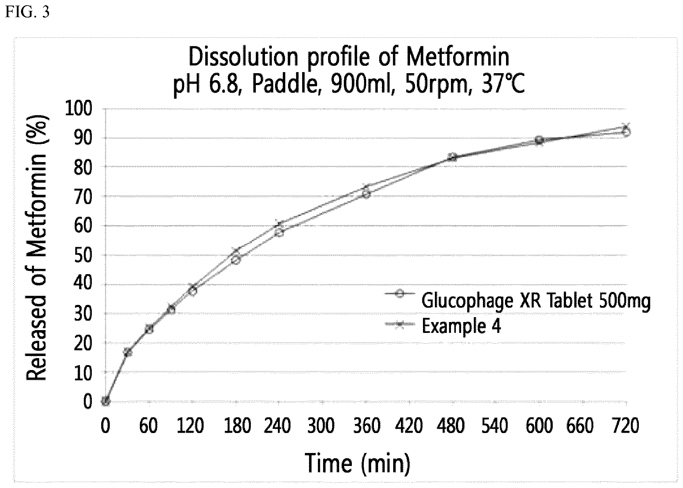

FIG. 3 is a graph illustrating the results of a release experiment on a sustained-release formulation of metformin hydrochloride prepared in Example 4 of the present invention and 500 mg Glucophage XR tablets.

### Best Mode

Hereinafter, the present invention will be described in more detail with reference to the following Examples.

### Comparative Examples 1 to 4: Confirmation of the delaying effect in release by a swellable polymer

In order to prepare a gastric-retention type sustained-release formulation, swellable polymers, which are effective in sustained release of hydrophilic drugs, were mixed with metformin hydrochloride, tableted, and tested for their release rate and degree of swelling.

### (1) Preparation of metformin hydrochloride samples using various swellable polymers

In order to prepare a metformin granulated material according to the kinds of swellable polymers, the ingredients measured according to the composition shown in Table 1 were passed through a 20 mesh sieve, mixed, and then tableted at a hardness of 25±5 kp to obtain white tablets (720 mg/unit).

**Table 1**

| | Comp. Ex. 1 | Comp. Ex. 2 | Comp. Ex. 3 | Comp. Ex. 4 |
|---|---|---|---|---|
| Metformin Hydrochloride | 500 mg | 500 mg | 500 mg | 500 mg |
| Colloidal Silicon Dioxide | 10 mg | 10 mg | 10 mg | 10 mg |
| Polyethylene Oxide | 200 mg | - | - | - |
| Hypromellose | - | 200 mg | - | - |
| Sodium Alginate | - | - | 200 mg | - |
| Xanthan Gum | - | - | - | 200 mg |
| Magnesium Stearate | 10 mg | 10 mg | 10 mg | 10 mg |
| Total Weight | 720 mg | 720 mg | 720 mg | 720 mg |

### (2) Release of metformin drug

The formulations prepared in Comparative Examples 1 to 4 were tested in 900 mL of a release solution of phosphate butter (pH 6.8) at 37°C at 50 rpm according to the release method (Method II) described in USP, and the released solution was collected at the scheduled time and their respective release rates were analyzed via HPLC. The results are shown in Table 2 below.

The conditions used for HPLC are as follows.
- column: Phenomenex Luna (CI8, 250 mm × 4.6 mm, 5 µm)
- detector: spectrophotometric detector (228 nm)
- mobile phase: a mixed solution of NaH₂PO₄: purified water : ACN (1g:1L:1L), wherein the pH was adjusted to 3.0 with phosphoric acid
- flow rate: 1.0 mL/min
- column temperature: 30°C
- time of analysis: 5 min

**Table 2**

| Release Time (min) | Release Rate of Metformin Hydrochloride (%) | | | |
|---|---|---|---|---|
| | Comp. Ex. 1 | Comp. Ex. 2 | Comp. Ex. 3 | Comp. Ex. 4 |
| 30 | 20.9±1.6 | 23.5±0.3 | 23.2±1.7 | 26.5±0.2 |
| 60 | 32.4±2.6 | 35.1±0.4 | 37.5±2.2 | 38.3±0.4 |
| 90 | 41.7±3.4 | 44.1±0.6 | 49.0±2.3 | 47.1±0.4 |
| 120 | 49.7±4.0 | 51.7±0.3 | 59.1±2.9 | 54.9±0.5 |
| 180 | 62.6±4.9 | 63.7±0.6 | 74.3±1.7 | 65.7±0.5 |
| 240 | 72.6±5.1 | 73.1±0.6 | 84.1±0.8 | 74.2±0.4 |
| 300 | 87.3±4.2 | 86.0±0.5 | 95.4±0.3 | 87.9±0.7 |
| 360 | 95.2±2.5 | 93.5±0.5 | 99.6±0.2 | 96.5±0.5 |

As a result, as shown in Table 2, Comparative Examples 1 and 2, in which relatively high viscosity swellable polymers, polyethylene oxide (Polyox® WSR301; Colorcon, USA) and hypromellose (Hypromellose 90SH-100,000SR®; Shin-EtSu, JAPAN) were used, showed a more effective control on the release of metformin drug. When the drug release rate was compared between the two polymers according to time, the initial release rate was lower in Comparative Example 1, but the release rate at a later stage was lower in Comparative Example 2.

Polyethylene oxide, due to its relatively high hydrophilicity, interacts well with water in an aqueous phase. Therefore, water rapidly penetrates into the tablet and induces gelation, thereby effectively controlling the initial release rate. However, the rapidly gelated tablet undergoes a weakening of crosslinking as time goes by, thus resulting in a loose structure among particles. Accordingly, the release rate at a later stage appears to be relatively not well controlled.

In contrast, hypromellose is slowly gelated in an aqueous solution, and thus the initial release control is not satisfiable. However, once formed into hydrogel, it has a dense network structure, and thus appears to effectively control the release rate at a later stage.

### (3) Test for the degree of swelling

In order to select a swellable polymer suitable for the gastric-retention type sustained-release formulation, the swelling rates of the tablets prepared in Comparative Examples 1 to 4 were examined. For measurement of swelling rates, the tablets prepared in Comparative Examples 1 to 4 were fully soaked in distilled water and their volumes were measured at 2 hours, 4 hours, 6 hours, and 12 hours. For volume measurement, 10 mL of distilled water was added into a 20 mL graduated cylinder and then charged with two swollen tablets, and the increase in graduation was measured. The results are shown in Table 3.

**Table 3**

| Time (hr) | Volume Expansion (mL) | | | |
|---|---|---|---|---|
| | Comp. Ex. 1 | Comp. Ex. 2 | Comp. Ex. 3 | Comp. Ex. 4 |
| 0 | 1.2 | 1.2 | 1.2 | 1.2 |
| 2 | 3.6 | 3.0 | 1.8 | 2.4 |
| 4 | 4.8 | 4.2 | 2.8 | 3.2 |
| 6 | 5.6 | 5.0 | 3.2 | 3.6 |
| 12 | 6.6 | 5.8 | 3.8 | 4.2 |

According to the test results of swelling rate shown in Table 3, the samples of Comparative Examples 1 and 2, which showed an effective control of metformin drug release, showed an at least 4-fold higher swelling rate during the 6 hour period. Additionally, although it is important that the gastric-retention type sustained-release formulation swells within a short time period and becomes larger than the pylorus so as not to pass therethrough and to be retained in the stomach, it is also essential that the gastric-retention type sustained-release formulation maintains its shape for a certain period of time, and all the tablets used in the test were shown to maintain their shape for at least 12 hours.

### Comparative Examples 5 to 7, Example 1: Confirmation of the delaying effect in release due to water-insoluble coating

In Comparative Examples 1 to 4, it was confirmed that drug release can be controlled using a swellable polymer, and also that the swellable polymer has an expansion rate suitable for the gastric-retention type sustained-release formulation. However, since it is necessary to effectively control drug release using a small dose to provide convenience of administration for metformin formulations, which require large daily dose, a complex release mechanism utilizing various sustained-release agents was attempted. Accordingly, in order to select excipients effective in sustaining drug release via water-insoluble coating along with the swellable polymer, samples were prepared according to the method described below and then subjected to release tests.

### (1) Preparation of metformin hydrochloride samples according to water-insoluble coating

In order to control the release rate of metformin hydrochloride drug and maintain its concentration in the blood at a constant level, granules were prepared according to the compositions shown in Table 4 and tableted at a hardness of 25±5 kp to obtain white tablets (770 mg/unit).

Specifically, in Comparative Example 5, granules were prepared by adding a binding solution, which was prepared by dissolving ammonio methacrylate copolymer (Eudragit® RS PO; Evonik, USA) in a mixed solvent of isopropyl alcohol, acetone, and purified water, to a mixture of metformin hydrochloride and colloidal silicon dioxide (Aerosil® 200; Evonik, USA). The thus-prepared granules were passed through a 20 mesh sieve, and mixed with polyethylene oxide and magnesium stearate to obtain metformin hydrochloride granules of Comparative Example 5.

In Comparative Example 6, granules were prepared by adding a binding solution, which was prepared by dissolving ethyl cellulose (Ethocel®; Colorcon, USA) in a mixed solvent of ethanol and methylene chloride, to a mixture of metformin hydrochloride and colloidal silicon dioxide. The sifting and mixing processes were performed in the same manner as in Comparative Example 5.

In Comparative Example 7, granules were prepared by adding a binding solution, which was prepared by dissolving cetanol (Crodacol® C90; Croda, USA) in a mixed solvent of ethanol and purified water, to a mixture of metformin hydrochloride and colloidal silicon dioxide. The sifting and mixing processes were performed in the same manner as in Comparative Example 5.

In Example 1, as in Comparative Example 5, a binding solution, which was prepared by dissolving ammonio methacrylate copolymer in a mixed solvent of isopropyl alcohol, acetone, and purified water, was used. However, the binding solution was added to a mixture of metformin hydrochloride, colloidal silicon dioxide and polyethylene oxide to be granulated. That is, granules were obtained by coating both the swellable polymer and metformin hydrochloride together with a water-insoluble polymer. The thus-prepared granules were passed through a 20 mesh sieve and mixed with magnesium stearate to obtain a sustained-release formulation.

**Table 4**

| | Comp. Ex. 5 | Comp. Ex. 6 | Comp. Ex. 7 | Comp. Ex. 8 |
|---|---|---|---|---|
| Metformin Hydrochloride | 500 mg | 500 mg | 500 mg | 500 mg |
| Colloidal Silicon Dioxide | 10 mg | 10 mg | 10 mg | 10 mg |
| Polyethylene Oxide | 200 mg | 200 mg | 200 mg | 200 mg |
| Ammonio Methacrylate Copolymer | 50 mg | - | - | 50 mg |
| Ethylcellulose | - | 50 mg | - | - |
| Cetanol | - | - | 50 mg | - |
| Magnesium Stearate | 10 mg | 10 mg | 10 mg | 10 mg |
| Total Weight | 770 mg | 770 mg | 770 mg | 770 mg |

### (2) Release of metformin

The formulations prepared above were tested and analyzed by the same release test method used in Comparative Examples 1 to 4, and the results are shown in Table 5.

**Table 5**

| Release Time (min) | Release Rate of Metformin Hydrochloride (%) | | | |
|---|---|---|---|---|
| | Comp. Ex. 5 | Comp. Ex. 6 | Comp. Ex. 7 | Ex. 1 |
| 30 | 16.5±1.1 | 18.7±1.9 | 19.7±1.3 | 15.8±0.9 |
| 60 | 28.8±1.2 | 30.6±1.9 | 30.8±1.2 | 26.3±0.8 |
| 90 | 36.4±1.2 | 40.7±2.1 | 38.3±1.4 | 33.9±0.6 |
| 120 | 43.5±1.1 | 48.0±2.5 | 46.4±1.1 | 40.3±0.3 |
| 180 | 54.9±1.3 | 60.2±2.7 | 58.5±1.0 | 51.4±0.1 |
| 240 | 64.5±1.2 | 70.9±3.8 | 68.1±1.1 | 60.8±0.7 |
| 360 | 78.7±0.9 | 84.4±2.5 | 82.1±1.2 | 75.6±1.7 |
| 480 | 86.6±0.7 | 91.4±2.4 | 88.8±1.3 | 84.7±2.1 |
| 600 | 91.2±0.7 | 94.6±1.8 | 91.6±0.8 | 90.3±0.6 |
| 720 | 95.7±0.4 | 97.1±1.6 | 95.9±0.7 | 93.2±1.0 |

As a result, as shown in Table 5, the tablets including granulated material coated with a water-insoluble polymer (50 mg/unit tablet) tended to show a more controlled drug release, compared to the release rates of those in Comparative Examples 1 to 4, which were prepared using only swellable polymers. In particular, the tablets of Example 1, which were prepared by coating the swellable polymer and metformin hydrochloride together with a water-insoluble polymer, showed a lower release rate compared to those of Comparative Example 5 having the same amount of drug, due to the difference in preparation methods. This is because, although a water-insoluble film controls the release of drugs, when a hydrophilic swellable polymer is used in a later mixing according to the method of Comparative Example 5, water rapidly penetrates into the inside of the tablets to thereby cause reduction of the effect of the water-insoluble coating film. Accordingly, the method of Example 1, wherein a water-insoluble film was prepared by mixing the swellable polymer and metformin hydrochloride together for effective control of drug release, is preferable as a method for preparing a sustained-release formulation applying a dual control mechanism of drug release.

### Examples 2 to 4: Effect of using superdisintegrants

In Example 1, in order to maintain a constant concentration of metformin hydrochloride drug in the blood by controlling its release rate, the swellable polymer and metformin hydrochloride were coated together with a water-insoluble polymer, and thereby metformin hydrochloride granules were prepared.

The sustained-release granulated material according to Example 1 can effectively control drug release by forming a water-insoluble polymer film on the exterior of a swellable polymer, but as a result, it delays hydration of the swellable polymer. To remedy this problem, the initial drug expansion was improved through adjustment of wettability of formulations by mixing a superdisintegrant to the sustained-release granulated material.

### (1) Preparation of metformin hydrochloride samples according to the amount of superdisintegrants

The formulations in Examples 2 to 5 were prepared such that granules were prepared first in the same manner as in Example 1, and then at a later mixing, the granules were mixed with croscarmellose sodium or crospovidone and magnesium stearate according to the compositions shown in Table 6.

**Table 6**

| | Ex. 2 | Ex. 3 | Ex. 4 | Ex. 5 |
|---|---|---|---|---|
| Metformin Hydrochloride | 500 mg | 500 mg | 500 mg | 500 mg |
| Colloidal Silicon dioxide | 10 mg | 10 mg | 10 mg | 10 mg |
| Polyethylene Oxide | 200 mg | 200 mg | 200 mg | 200 mg |
| Ammonio Methacrylate Copolymer | 50 mg | 50 mg | 50 mg | 50 mg |
| Croscarmellose Sodium | 10 mg | 25 mg | 40 mg | - |
| Crospovidone | - | - | - | 25 mg |
| Magnesium Stearate | 10 mg | 10 mg | 10 mg | 10 mg |
| Total Weight | 780 mg | 795 mg | 810 mg | 795 mg |

### (2) Release of metformin

The formulations prepared above were tested and analyzed by the same release test method used in Comparative Examples 1 to 5, and the results are shown in Table 7.

**Table 7**

| Release Time (min) | Release Rate of Metformin Hydrochloride (%) | | | | |
|---|---|---|---|---|---|
| | Ex. 2 | Ex. 3 | Ex. 4 | Ex. 5 | Glucophage XR Tablet 500 mg |
| 30 | 17.4±1.4 | 16.5±0.2 | 16.9±1.6 | 16.8±2.1 | 16.8±0.8 |
| 60 | 26.2±1.8 | 25.4±0.9 | 25.3±2.0 | 25.8±2.2 | 24.7±0.1 |
| 90 | 32.5±1.0 | 32.2±1.0 | 32.4±2.3 | 32.7±2.1 | 31.5±0.1 |
| 120 | 39.9±0.9 | 39.0±0.6 | 39.3±2.7 | 39.5±2.5 | 37.8±0.2 |
| 180 | 50.7±1.0 | 50.7±0.3 | 51.6±2.0 | 52.0±1.7 | 48.4±0.3 |
| 240 | 59.7±0.8 | 60.8±0.9 | 60.8±1.8 | 61.0±1.4 | 57.8±0.6 |
| 360 | 74.4±1.3 | 74.7±0.7 | 73.4±1.3 | 75.5±1.4 | 70.7±0.2 |
| 480 | 84.0±0.7 | 85.6±0.5 | 83.2±1.9 | 86.5±1.4 | 83.5±1.0 |
| 600 | 89.5±0.8 | 89.4±0.5 | 88.2±1.7 | 90.1±1.4 | 89.3±0.2 |
| 720 | 93.0±1.2 | 94.4±0.4 | 93.7±1.6 | 95.2±1.1 | 91.9±0.7 |

As a result, as shown in Table 7, it was confirmed that there was a slight decrease or no change in release rate by the effects of croscarmellose sodium and crospovidone, which were used as superdisintegrants.

### (3) Test for the degree of swelling

The formulations prepared above were tested and analyzed by the same swelling test method used in Comparative Examples 1 to 5, and the results are shown in Table 8.

**Table 8**

| Time (hr) | Volume Expansion (mL) | | | | | |
|---|---|---|---|---|---|---|
| | Ex. 1 | Ex. 2 | Ex. 3 | Ex. 4 | Ex. 5 | Glucophage XR Tablet 500 mg |
| 0 | 1.3 | 1.3 | 1.3 | 1.3 | 1.3 | 1.8 |
| 2 | 3.9 | 4.0 | 4.2 | 4.3 | 4.2 | 4.2 |
| 4 | 5.2 | 5.3 | 5.4 | 5.5 | 5.4 | 5.4 |
| 6 | 6.1 | 6.1 | 6.3 | 6.4 | 6.3 | 6.6 |
| 12 | 7.2 | 7.2 | 7.3 | 7.4 | 7.3 | 8.2 |

According to the test results of swelling rate shown in Table 8, the swelling rates according to using croscarmellose sodium or crospovidone as a superdisintegrant were similar to each other, and the volume expansion rate increased along with the increase in its amount. There was a difference at the initial volume among the tablets prepared in Examples because the masses of these tablets corresponded to about 80% of those of 500 mg Glucophage XR tablets, whose tablet mass was above 1,000 mg. However, due to their high swelling rates, they were shown to have expanded to have volumes similar to or even greater than those from the 2 hour time point and thereafter.

## Claims

1. A gastric-retention type sustained-release formulation comprising:
a sustained-release granulated material, which comprises granules comprising metformin or a pharmaceutically acceptable salt thereof and a swellable polymer, and a coating layer containing a water-insoluble polymer applied on the surface of the granules; and
a superdisintegrant,
wherein the superdisintegrant is present as an extragranular component mixed with the sustained-release granulated material,
wherein the superdisintegrant is at least one selected from the group consisting of croscarmellose sodium and crospovidone, and
wherein the superdisintegrant is comprised in the amount from 0.5 wt% to 5 wt% based on the total amount of the formulation.

2. The sustained-release formulation of claim 1, wherein the metformin or the pharmaceutically acceptable salt thereof is comprised in the amount from 250 mg to 1000 mg.

3. The sustained-release formulation of claim 1, wherein the swellable polymer is at least one selected from the group consisting of hypromellose, hydroxyethyl cellulose, hydroxypropyl cellulose, polyethylene oxide, carrageenan, natural gum, guar gum, tragacanth, acacia gum, locust bean gum, xanthan gum, alginate, polyvinyl alcohol, and polyvinylpyrrolidone.

4. The sustained-release formulation of claim 3, wherein the swellable polymer is at least one selected from the group consisting of hypromellose and polyethylene oxide.

5. The sustained-release formulation of claim 3, wherein the swellable polymer has a viscosity of 100 mPas or higher.

6. The sustained-release formulation of claim 3, wherein the swellable polymer is comprised in the amount from 10 wt% to 40 wt% based on the total amount of the formulation

7. The sustained-release formulation of claim 1, wherein the water-insoluble polymer is at least one selected from the group consisting of methacrylic acid copolymer, ethylcellulose, cellulose acetate succinate, cellulose acetate phthalate, fatty acid, fatty acid ester, fatty acid alcohol, and wax.

8. The sustained-release formulation of claim 7, wherein the water-insoluble polymer is at least one selected from the group consisting of methacrylic acid copolymer and ethylcellulose.

9. The sustained-release formulation of claim 7, wherein the water-insoluble polymer is comprised in the amount from 1 wt% to 20 wt% based on the total amount of the formulation.

10. The sustained-release formulation of claim 1, wherein the superdisintegrant is croscarmellose sodium.

11. The sustained-release formulation of claim 1, wherein the sustained-release formulation is a plain tablet, a film-coated tablet, a single-layer tablet, a double-layer tablet, a multi-layer tablet, or a cored tablet.

12. A method of preparing the sustained-release formulation of claim 1, comprising:
(a) preparing granules comprising metformin or a pharmaceutically acceptable salt thereof; and a swellable polymer;
(b) preparing a sustained-release granulated material, wherein a coating layer is formed thereon by coating the granules with a water-insoluble polymer; and
(c) preparing a formulation by mixing the sustained-release granulated material and a superdisintegrant.

13. The method of claim 12, further comprising forming a film layer on the external surface of the sustained-release formulation.

## Patentansprüche

1. Formulierung mit verzögerter Freisetzung vom Typ der gastrischen Retention, die aufweist:
granuliertes Material mit verzögerter Freisetzung, das Körnchen aufweist, welche Metformin oder ein pharmazeutisch akzeptierbares Salz davon aufweisen, und ein quellfähiges Polymer, und eine Beschichtungsschicht, die ein wasserunlösliches Polymer enthält und auf die Oberfläche der Körnchen aufgebracht ist; und
ein Superzerfallsmittel,
wobei das Superzerfallsmittel als extragranuläre Komponente vorhanden ist, die mit dem granulierten Material mit verzögerter Freisetzung gemischt ist,
wobei das Superzerfallsmittel wenigstens eines ist, das ausgewählt wird aus der Gruppe bestehend aus Croscarmellose-Natrium und Crospovidon, und
wobei das Superzerfallsmittel in der Menge von 0,5 Gew.-% bis 5 Gew.-% basierend auf der Gesamtmenge der Formulierung enthalten ist.

2. Formulierung mit verzögerter Freisetzung nach Anspruch 1, wobei das Metformin oder das pharmazeutisch akzeptierbare Salz davon in der Menge von 250 mg bis 1000 mg enthalten ist.

3. Formulierung mit verzögerter Freisetzung nach Anspruch 1, wobei das quellfähige Polymer wenigstens eines ist, das ausgewählt wird aus der Gruppe bestehend aus Hypromellose, Hydroxyethylcellulose, Hydroxypropylcellulose, Polyethylenoxid, Carrageen, Naturgummi, Guargummi, Tragant, Akaziengummi, Johannisbrotkernmehl, Xanthangummi, Alginat, Polyvinylalkohol und Polyvinylpyrrolidon.

4. Formulierung mit verzögerter Freisetzung nach Anspruch 3, wobei das quellfähige Polymer wenigstens eines ist, das ausgewählt wird aus der Gruppe bestehend aus Hypromellose und Polyethylenoxid.

5. Formulierung mit verzögerter Freisetzung nach Anspruch 3, wobei das quellfähige Polymer eine Viskosität von 100 mPas oder höher hat.

6. Formulierung mit verzögerter Freisetzung nach Anspruch 3, wobei das quellfähige Polymer in der Menge von 10 Gew.-% bis 40 Gew.-% basierend auf der Gesamtmenge der Formulierung enthalten ist.

7. Formulierung mit verzögerter Freisetzung nach Anspruch 1, wobei das wasserunlösliche Polymer wenigstens eines ist, das ausgewählt wird aus der Gruppe bestehend aus Methacrylsäure-Copolymer, Ethylcellulose, Celluloseacetatsuccinat, Celluloseacetatphthalat, Fettsäure, Fettsäureester, Fettsäurealkohol und Wachs.

8. Formulierung mit verzögerter Freisetzung nach Anspruch 7, wobei das wasserunlösliche Polymer wenigstens eines ist, das ausgewählt wird aus der Gruppe bestehend aus Methacrylsäure-Copolymer und Ethylcellulose.

9. Formulierung mit verzögerter Freisetzung nach Anspruch 7, wobei das wasserunlösliche Polymer in der Menge von 1 Gew.-% bis 20 Gew.-% basierend auf der Gesamtmenge der Formulierung enthalten ist.

10. Formulierung mit verzögerter Freisetzung nach Anspruch 1, wobei das Superzerfallsmittel Croscarmellose-Natrium ist.

11. Formulierung mit verzögerter Freisetzung nach Anspruch 1, wobei die Formulierung mit verzögerter Freisetzung eine flache Tablette, eine Filmtablette, eine Einschichttablette, eine Zweischichttablette, eine Mehrschichttablette oder eine Tablette mit Kern ist.

12. Verfahren zum Herstellen der Formulierung mit verzögerter Freisetzung nach Anspruch 1, das aufweist:
(a) Herstellen von Körnchen, die Metformin oder ein pharmazeutisch akzeptierbares Salz davon aufweisen; und von einem quellfähigen Polymer;
(b) Herstellen eines granulierten Materials mit verzögerter Freisetzung, wobei eine Beschichtungsschicht durch Beschichten der Körnchen mit einem wasserunlöslichen Polymer darauf ausgebildet wird; und
(c) Herstellen einer Formulierung durch Mischen des granulierten Materials mit verzögerter Freisetzung und eines Superzerfallsmittels.

13. Verfahren nach Anspruch 12, das des Weiteren das Ausbilden einer Filmschicht auf der äußeren Oberfläche der Formulierung mit verzögerter Freisetzung aufweist.

## Revendications

1. Formulation à libération prolongée de type à rétention gastrique comprenant :
un matériau granulé à libération prolongée, qui comprend des granules comprenant de la metformine ou un sel pharmaceutiquement acceptable de celle-ci et un polymère gonflable, et une couche de revêtement contenant un polymère insoluble dans l'eau appliqué sur la surface des granules ; et
un super-désintégrant,
dans laquelle le super-désintégrant est présent sous la forme d'un composé extra-granulaire mélangé au matériau granulé à libération prolongée,
dans laquelle le super-désintégrant est au moins un élément sélectionné dans le groupe constitué de la croscarmellose sodique et de la crospovidone, et
dans laquelle le super-désintégrant est compris en une quantité de 0,5 % en poids à 5 % en poids sur la base de la quantité totale de la formulation.

2. Formulation à libération prolongée selon la revendication 1, dans laquelle la metformine ou le sel pharmaceutiquement acceptable de celle-ci est compris(e) dans une quantité de 250 mg à 1000 mg.

3. Formulation à libération prolongée selon la revendication 1, dans laquelle le polymère gonflable est au moins un élément sélectionné dans le groupe constitué de l'hypromellose, de l'hydroxyéthyl cellulose, de l'hydroxypropyl cellulose, de l'oxyde de polyéthylène, de la carraghénane, de la gomme naturelle, de la gomme de guar, de la tragacanthe, de la gomme d'acacia, de la gomme de caroubier, de la gomme de xanthane, de l'alginate, de l'alcool polyvinylique, et de la polyvinyle pyrrolidone.

4. Formulation à libération prolongée selon la revendication 3, dans laquelle le polymère gonflable est au moins un élément sélectionné dans le groupe constitué de l'hypromellose et de l'oxyde de polyéthylène.

5. Formulation à libération prolongée selon la revendication 3, dans laquelle le polymère gonflable a une viscosité de 100 mPas ou plus.

6. Formulation à libération prolongée selon la revendication 3, dans laquelle le polymère gonflable est compris dans une quantité de 10 % en poids à 40 % en poids sur la base de la quantité totale de la formulation.

7. Formulation à libération prolongée selon la revendication 1, dans laquelle le polymère insoluble dans l'eau est au moins un élément sélectionné dans le groupe constitué d'un copolymère d'acide méthacrylique, de l'éthylcellulose, de l'acétate succinate de cellulose, de l'acétate phtalate de cellulose, d'un acide gras, d'un ester d'acide gras, d'un alcool d'acide gras, et d'une cire.

8. Formulation à libération prolongée selon la revendication 7, dans laquelle le polymère insoluble dans l'eau est au moins un élément sélectionné dans le groupe constitué d'un copolymère d'acide méthacrylique et de l'éthyl cellulose.

9. Formulation à libération prolongée selon la revendication 7, dans laquelle le polymère insoluble dans l'eau est compris dans une quantité de 1 % en poids à 20 % en poids sur la base de la quantité totale de la formulation.

10. Formulation à libération prolongée selon la revendication 1, dans laquelle le super-désintégrant est la croscarmellose sodique.

11. Formulation à libération prolongée selon la revendication 1, dans laquelle la formulation à libération prolongée est un comprimé ordinaire, un comprimé pelliculé, un comprimé monocouche, un comprimé bicouche, un comprimé multicouche, ou un comprimé à noyau.

12. Procédé de préparation de la formulation à libération prolongée selon la revendication 1, comprenant :
(a) la préparation de granules comprenant de la metformine ou un sel pharmaceutiquement acceptable de celle-ci ; et d'un polymère gonflable ;
(b) la préparation d'un matériau granulé à libération prolongée, dans lequel une couche de revêtement est formée sur celui-ci par revêtement des granules avec un polymère insoluble dans l'eau ; et
(c) la préparation d'une formulation par mélange du matériau granulé à libération prolongée et d'un super-désintégrant.

13. Procédé selon la revendication 12 comprenant en outre la formation d'une couche de film sur la surface externe de la formulation à libération prolongée.
